# FASCICULE DE BREVET EUROPEEN

(11) **EP 4 072 701 B1**
(45) Date de publication et mention de la délivrance du brevet: **12.06.2024**
(21) Numéro de dépôt: 20816470.7
(22) Date de dépôt: 01.12.2020
(51) Int. Cl.: B01D 53/14, B01D 53/26, C10L 3/10

(54) **PROCEDE DE REGENERATION D'UNE SOLUTION AQUEUSE DE MEG CONTENANT DES SELS AVEC TRAITEMENT DE LA PURGE**
VERFAHREN ZUR REGENERIERUNG EINER WÄSSRIGEN LÖSUNG VON MEG-HALTIGEN SALZEN MIT EINER SPÜLBEHANDLUNG
METHOD FOR REGENERATING AN AQUEOUS SOLUTION OF MEG CONTAINING SALTS WITH PURGE TREATMENT

(30) Priorité: 13.12.2019 FR 1914427
(43) Date de publication de la demande: 19.10.2022
(73) Titulaire: IFP Energies nouvelles, 92852 Rueil-Malmaison Cedex (FR); Axens, 92500 Rueil Malmaison (FR)
(72) Inventeur: DELFORT, Bruno, 92852 Rueil-Malmaison Cedex (FR); WENDER, Aurélie, 92852 Rueil-Malmaison Cedex (FR); MULLER, Elsa, 92852 Rueil-Malmaison Cedex (FR); COURTIAL, Xavier, 92500 Rueil-Malmaison (FR); PARER, Guillaume, 92500 Rueil-Malmaison (FR); LANFREY, Pierre-Yves, 92500 Rueil-Malmaison (FR)
(74) Mandataire: IFP Energies nouvelles
(86) Numéro de dépôt international: PCT/EP2020/084179
(87) Numéro de publication internationale: WO 2021/115852

(56) Documents cités:
- EP-A1- 2 860 168
- WO-A1-2007/073204
- FR-A1- 2 743 069
- FR-A1- 2 846 323
- US-A1- 2015 119 609
- US-A1- 2017 129 835
- US-A1- 2018 029 963

## Description

### Domaine technique

La présente invention concerne le domaine de la régénération d'une solution aqueuse de monoéthylène glycol (MEG) contenant des sels dissous utilisé pour le transport du gaz naturel.

### Technique antérieure

Le gaz naturel en sortie des puits de production est souvent associé à de l'eau de gisement contenant des sels dissous (chlorure de sodium, chlorure de potassium, chlorure de calcium, bicarbonate de sodium, etc. ). Le gaz naturel est transporté du lieu de production vers un lieu de traitement par circulation dans des conduites. En fonction des conditions de transport, et notamment de la pression et de la température, il est possible que l'eau contenue dans le gaz naturel forme des bouchons d'hydrates pouvant conduire à l'arrêt de la production.

Pour éviter ces problèmes, un inhibiteur d'hydrates est généralement injecté dans les conduites de transport. Le MEG, appelé aussi éthane-1,2-diol ou éthylène glycol, est couramment utilisé comme inhibiteur d'hydrates dans le transport du gaz naturel, souvent saturé en eau, du lieu de production au lieu de traitement. Le MEG est classiquement injecté au niveau des puits de forage pour sécuriser le transport du gaz naturel jusqu'aux unités de traitement. Pour ce faire, une solution aqueuse contenant entre 70% et 95% poids de MEG, connue sous le nom de MEG purifié ou « Lean MEG » en anglais, est utilisée.

Une fois séparée des condensats et du gaz naturel, la phase aqueuse récupérée forme une solution contenant généralement entre 15% et 60 % poids de MEG, appelée « Rich MEG » en anglais ou solution de « MEG riche ». La solution de MEG riche est composée du MEG purifié injecté en tête de puit, d'eau de la formation souterraine et des sels provenant de cette formation, d'eau issue de la condensation du gaz et de traces d'hydrocarbures dissous et libres. La solution de MEG riche nécessite ainsi d'être régénérée et dessalée afin de pouvoir être réutilisée pour son injection en tête de puit de forage. Le procédé permettant la régénération et le dessalage est généralement appelé selon le terme générique « MEG reclaiming ».

Le MEG entre dans le procédé de reclaiming avec des impuretés telles que par exemple de l'eau, des hydrocarbures, des sels inorganiques comme par exemple le chlorure de sodium ou le chlorure de potassium, et des sels dérivés d'acides carboxyliques tels par exemple les formiates, les acétates, les propionates, les butyrates, les oxalates et les glycolates de sodium et de potassium. En sortie de procédé, le MEG a été régénéré/dessalé et se présente sous la forme d'un mélange MEG/eau réinjectable en tête de puits de forage, qui est la solution aqueuse comportant entre 70% et 95% poids de MEG dite de MEG purifié (« Lean MEG »), aussi appelé dans la présente description solution de MEG régénérée.

Des systèmes de distillation du MEG pour séparer le MEG du mélange eau et MEG sont connus de l'homme du métier, ces procédés étant qualifiés de procédés de régénération de MEG. En général, les systèmes de l'art antérieur permettent d'obtenir une solution aqueuse contenant entre 70% et 95% poids de MEG.

Par ailleurs, si les sels ne sont pas retirés de la solution de MEG riche, la régénération du MEG conduit à concentrer et accumuler les sels, provenant principalement de l'eau de la formation souterraine, dans la solution de MEG régénérée et dans les boucles de recycle du procédé de régénération. L'accumulation de ces sels est à l'origine de nombreux problèmes opératoires, comme par exemple des phénomènes de bouchage des installations de régénération du MEG pouvant aller jusqu'à provoquer leur arrêt. C'est la raison pour laquelle les procédés de régénération du MEG peuvent comporter en plus de la séparation de l'eau du MEG une étape de dessalage du MEG, en particulier dans des procédés opérant à des pressions inférieures à la pression atmosphérique.

La solution de MEG riche, contenant de l'eau et des sels dissous, comporte ainsi :
- des sels qui précipitent dans des conditions compatibles avec les installations de régénération et dessalage de solutions de MEG riche, tel que les sels inorganiques comme le chlorure de sodium, chlorure de potassium, chlorure de calcium, bicarbonate de sodium, etc. ;
- des sels qui ne précipitent pas dans des conditions compatibles avec les installations de régénération et dessalage de solutions de MEG riche, tel que les sels organiques, comme les sels d'acides carboxyliques de type formate, l'acétate, le propionate, le butyrate, etc., issus du gaz naturel et/ou de l'eau de la formation souterraine, et/ou de la dégradation du MEG.

Les sels d'acides carboxyliques sont extrêmement solubles dans les solutions aqueuses de MEG, et ne précipitent pas à faible concentration, à la différence des sels inorganiques. Ils peuvent être également présent dans la solution aqueuse de MEG sous forme d'acides carboxyliques, qui sont volatils, selon le pH de la solution aqueuse de MEG considéré. Au contraire, sous forme de sels, notamment de sels alcalins, alcalinoterreux ou métalliques, les sels d'acides organiques ne sont pas volatils.

Actuellement, la plupart des procédés de régénération et dessalage de solutions de MEG riches permettent d'éliminer une grande partie de l'eau par distillation et les sels inorganiques par précipitation.

En revanche, l'élimination des sels d'acides carboxyliques reste problématique.

Les sels d'acides organiques, notamment les sels d'acides carboxyliques , une fois solubilisés, ont tendance à s'accumuler dans les solutions de MEG, notamment au niveau des boucles de recycle des unités de vaporisation des installations de régénération et dessalage. Plus il y a de sels organiques solubilisés/accumulés dans la solution de MEG, plus la viscosité de la solution augmente. En cela, lorsqu'ils s'accumulent dans le procédé, les sels d'acides organiques solubilisés peuvent provoquer des phénomènes de figeage du MEG et conduire ainsi à des problèmes opératoires, voire à l'arrêt de l'unité. L'énergie nécessaire à apporter lors de la régénération est également susceptible d'augmenter.

Il est ainsi nécessaire de contrôler la concentration en sels d'acides carboxyliques solubilisés dans les boucles de vaporisation du MEG au cours du fonctionnement du procédé afin de limiter l'augmentation de viscosité et d'éviter les phénomènes de bouchage des installations.

Il est connu de l'homme du métier la possibilité de contrôler la teneur en sels dans le procédé de régénération en utilisant une purge. Ladite purge permet d'évacuer hors de l'unité une partie des sels dissous et/ou en suspension en purgeant un flux comprenant des sels dissous et/ou en suspension ainsi que du MEG. La quantité de sels purgés est adaptée pour maintenir la concentration en sels solubilisés et/ou en suspension au cours du fonctionnement et donc éviter une augmentation de viscosité pouvant causer les phénomènes de bouchage des installations.

Par exemple le brevet EP1261410 décrit une installation et un procédé de purification d'inhibiteurs de formation d'hydrates tel que le MEG. La gestion des sels dissous dans la solution de MEG est réalisée grâce à une purge : le MEG à traiter est envoyé dans un ballon de flash ou une colonne opérant sous vide de façon à séparer un flux liquide en fond de ballon (ou colonne) comprenant du MEG et les sels, et en tête de ballon (ou colonne) un flux essentiellement gazeux comprenant l'eau et du MEG. Le flux de MEG et sels est en partie réchauffé pour être recyclé au ballon (ou colonne), l'autre partie est purgée, la quantité purgée dépendant de la concentration en sels. Cette purge permet de prévenir la saturation et la précipitation de sels dans le flux liquide de MEG et sels en sortie du ballon (ou colonne). Le flux de tête est distillé sous vide pour séparer l'eau et les gaz (en tête) et récupérer le MEG purifié (en fond) qui est recyclé sur le champ de gaz naturel. Le brevet US5993608 divulgue également la possibilité d'effectuer une purge similaire sur l'effluent liquide issu d'une zone de séparation vapeur/liquide recevant la solution de MEG.

Cependant, une telle purge conduit à une perte de MEG, ce qui réduit l'efficacité du procédé de régénération.

La présente invention porte sur un procédé visant à limiter la perte en MEG rencontrée lors de purges traditionnelles effectuées pour évacuer du système les sels d'acides organiques, et vise à limiter l'accumulation de ces sels dans l'installation.

Il est connu certaines méthodes de purification du MEG qui prennent en compte la problématique de l'accumulation des sels d'acides d'organiques dans la solution de MEG.

Ainsi, le brevet US9284244 décrit une méthode de précipitation et d'élimination de sels d'acides carboxyliques d'une solution de MEG qui consiste à forcer la précipitation de ces sels par ajout d'un antisolvant et aussi par addition d'une solution de sels inorganiques comprenant des cations divalents. Dans ce document, il est décrit que la performance est atteinte du fait de l'introduction de composés chimiques.

Le brevet US9732019 décrit une méthode pour stripper certains acides carboxyliques d'une solution de MEG riche, en mettant en contact la solution de MEG riche avec un gaz de stripage dans une colonne de stripage. Cette méthode concerne les acides carboxyliques et non leurs sels. Selon cette méthode, tout le flux de solution de MEG riche à régénérer est soumis au stripage.

D'ultérieurs procédés de l'art antérieur dont connus de WO 2007/073204 A1 et de FR 2 846 323 A1.

### Objectifs et Résumé de l'invention

La présente invention a pour objectif général de fournir un procédé de régénération et dessalage d'une solution de MEG riche permettant de récupérer un maximum de MEG pour former la solution régénérée tout en éliminant les sels inorganiques et au moins en partie les sels d'acides carboxyliques présents dans la solution.

La présente invention vise ainsi en particulier à éviter les effets néfastes d'une augmentation de la concentration en sels d'acides carboxyliques dissous dans la solution de MEG lors de sa régénération, en particulier une augmentation de viscosité importante pouvant conduire à des phénomènes de bouchage ou autres problèmes opératoires dans l'installation de régénération.

En particulier, la présente invention vise à remplir au moins l'un des objectifs suivants :
- éliminer au moins en partie, de préférence en totalité, les sels d'acides carboxyliques de la solution aqueuse de MEG lors des étapes du procédé de régénération tout en maximisant la quantité de MEG récupérée servant à former la solution de MEG régénérée ;
- éliminer les sels d'acides carboxyliques de la solution aqueuse de MEG lors des étapes du procédé de régénération tout en maximisant la quantité de MEG récupérée servant à former la solution de MEG régénérée, sans avoir recours à l'addition de composés ou de réactifs chimiques favorisant la précipitation des sels d'acides carboxyliques.

Il a ainsi été mis en évidence par les inventeurs qu'il était possible d'atteindre au moins l'un de ces objectifs en traitant la solution aqueuse de MEG riche par un procédé de régénération et dessalage comportant une suite d'opérations, notamment des opérations adaptées à l'élimination de chaque type de sels, i.e. les sels inorganiques et les sels d'acides carboxyliques, pour fournir une solution de MEG régénérée.

Ainsi, pour atteindre au moins l'un des objectifs susvisés, parmi d'autres, la présente invention propose, selon un premier aspect, un procédé de régénération d'une solution de MEG contenant de l'eau et des sels dissous, comportant les étapes suivantes :
a) on vaporise sous vide ladite solution dans une première unité de vaporisation pour produire un effluent gazeux comportant du MEG et de l'eau, et un résidu liquide enrichi en MEG et en sels dont une première partie est recyclée dans ladite première unité de vaporisation sous vide ;
b) éventuellement on envoie une deuxième partie dudit résidu liquide enrichi en MEG et en sels dans un réservoir dans lequel on baisse la température de ladite deuxième partie dudit résidu liquide enrichi en sels de manière à précipiter des sels inorganiques pour former un flux enrichi en sels précipités dont une première fraction est de préférence recyclée dans le réservoir et un flux appauvri en sels précipités recyclé dans l'unité de vaporisation sous vide à l'étape a) ;
c) on envoie, de préférence de manière intermittente, ladite deuxième partie dudit résidu liquide enrichi en MEG et en sels issue de l'étape a) ou une deuxième fraction dudit flux enrichi en sels précipités obtenu à l'étape b), dans une zone de séparation solide/liquide pour séparer un flux contenant des sels précipités comportant des sels inorganiques et un effluent liquide comportant des sels dissous parmi lesquels des sels d'acides carboxyliques;
d) on envoie, de préférence de manière intermittente, une portion (114) ou la totalité dudit effluent liquide (112) obtenu à l'étape c) dans une deuxième unité de vaporisation (1004) différente de la première unité de vaporisation sous vide (1000) pour opérer une séparation entre le MEG et au moins en partie les sels d'acides carboxyliques en vaporisant sous vide la portion (114) ou la totalité dudit effluent liquide (112) obtenu à l'étape c) pour former une vapeur enrichie en MEG et appauvrie en sels d'acides carboxyliques (115) et un effluent résiduel liquide enrichi en sels d'acides carboxyliques (116);
e) on recycle ladite la vapeur enrichie en MEG et appauvrie en sels d'acides carboxyliques à l'étape a), ou on mélange ladite la vapeur enrichie en MEG et appauvrie en sels d'acides carboxyliques avec l'effluent gazeux comportant du MEG et de l'eau issu de l'étape a), et on utilise ledit effluent gazeux comportant du MEG et de l'eau issu de l'étape a) ou ledit mélange pour produire la solution régénérée de MEG.

Selon une ou plusieurs mises en oeuvre, à l'étape e) le flux de MEG appauvri en sels d'acides carboxyliques ou en acides carboxyliques issu de l'étape d) est recyclé à l'étape a).

On peut effectuer en outre une étape f) de purification de l'effluent gazeux issu de l'étape a) dans une unité de purification pour former un flux d'eau et la solution régénérée de MEG, comportant de préférence de 70% à 95% poids de MEG.

Cette étape de purifications f) peut être une distillation sous vide, de préférence opérée à une pression comprise entre 0,01 MPa et 0,07 MPa.

Selon une ou plusieurs mises en oeuvre, préalablement à l'étape a), on réalise une vaporisation à pression atmosphérique de la solution de MEG contenant de l'eau et des sels dissous à régénérer dans une unité de vaporisation initiale, de préférence à une température comprise entre 100°C et 155°C et une pression comprise entre 0,1 MPa et 0,15 MPa, pour produire un effluent gazeux enrichi en eau et un résidu liquide enrichi en MEG et en sels dont la totalité est envoyée à l'étape a).

Selon une ou plusieurs mises en oeuvre, l'étape a) est opérée à une température comprise entre 120°C et 155°C, et une pression comprise entre 0,01 MPa et 0,07 MPa.

Selon une ou plusieurs mises en oeuvre, l'étape c) est opérée à une température comprise entre 50°C et 90°C et à pression atmosphérique, de préférence à une pression comprise entre 0,1 MPa et 0,15 MPa.

Selon une ou plusieurs mises en oeuvre, on envoie la deuxième fraction dudit flux enrichi en sels précipités issu du réservoir dans l'unité de séparation solide/liquide de manière intermittente, de préférence si le concentration en sels inorganiques dudit flux enrichi en sels précipités est comprise entre 10 % poids et 50 % poids, de préférence entre 15 % poids et 30 % poids.

Selon une ou plusieurs mises en oeuvre, on envoie de manière intermittente la portion ou la totalité dudit effluent liquide obtenu à l'étape c) dans l'unité de séparation de l'étape d).

Selon l'invention, à l'étape d) l'unité de séparation est une deuxième unité de vaporisation, et on vaporise sous vide la portion ou la totalité dudit effluent liquide obtenu à l'étape c) pour former une vapeur enrichie en MEG et appauvrie en sels d'acides carboxyliques et un effluent résiduel liquide enrichi en sels d'acides carboxyliques.

On peut envoyer un flux supplémentaire liquide ou gazeux dans ladite deuxième unité de vaporisation pour augmenter la quantité de MEG récupérée dans le flux lors de l'étape d), ledit flux étant choisi parmi l'eau, la vapeur d'eau, ou un gaz inerte.

Avantageusement ladite étape d) est opérée à une température comprise entre 120°C et 155°C, et une pression comprise entre 0,01 MPa et 0,07 MPa.

D'autres objets et avantages de l'invention apparaîtront à la lecture de la description qui suit d'exemples de réalisations particuliers de l'invention, donnés à titre d'exemples non limitatifs, la description étant faite en référence aux figures annexées décrites ci-après.

### Liste des figures

La figure 1 représente un schéma du procédé de régénération d'une solution de MEG selon un mode de réalisation de l'invention.
La figure 2 représente un schéma du procédé de régénération d'une solution de MEG selon un autre mode de réalisation de l'invention.

Sur les figures, les mêmes références désignent des éléments identiques ou analogues.

### Description des modes de réalisation

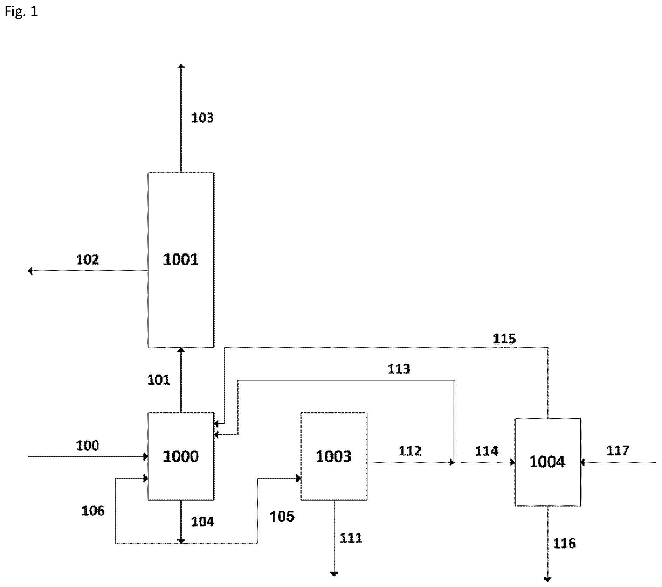

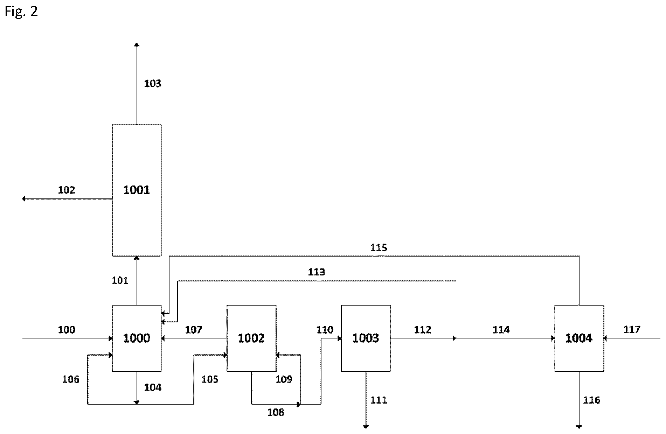

Deux modes de réalisation du procédé selon l'invention sont illustrés aux figures 1 et 2, et servent à la description ci-après du procédé pour une meilleure compréhension des étapes et des différents flux impliqués.

Ces illustrations du procédé selon l'invention ne comportent pas l'ensemble des composantes nécessaires à sa mise en oeuvre, par exemple les échangeurs de chaleur, pompes, mélangeurs etc. Seuls les éléments nécessaires à la compréhension de l'invention y sont représentés, l'homme du métier étant capable de compléter ces représentations pour mettre en oeuvre l'invention.

L'expression « compris(e) entre ... et ... » signifie que les valeurs limites de l'intervalle sont incluses dans la gamme de valeurs décrite, sauf précisé autrement.

Partout dans la description, la somme des fractions massiques exprimées en % poids des différents composés d'une solution est égale à 100 % en poids de ladite solution.

Les fractions massiques de MEG et d'eau d'un flux/effluent sont exprimées en % poids du flux/effluent hors sels, i.e. ne tenant pas compte des sels, sauf si spécifié autrement.

Dans la présente description, les étapes sont opérées en continu, à moins que leur fonctionnement intermittent ne soit précisé.

Dans la présente description, les pressions sont exprimées en valeurs absolues, sauf indiqué autrement.

Dans la présente invention, les différentes plages de paramètres pour une étape donnée tels que les plages de pression et les plages de température peuvent être utilisées seules ou en combinaison. Par exemple, dans la présente invention, une plage de valeurs préférées de pression peut être combinée avec une plage de valeurs de température plus préférée.

Dans la présente description, les termes « enrichi(e) » et « appauvri » en un ou plusieurs composés dans un effluent/flux sortant à un étape doivent s'entendre relativement à la concentration en ledit ou lesdits composés dans un effluent/flux entrant. Ainsi un flux sortant enrichi en MEG signifie que le flux sortant a une concentration en MEG plus élevée que la concentration en MEG du flux entrant. De la même manière un flux sortant appauvri en sels contient une concentration en sels moins élevée que la concentration en sels du flux entrant.

Selon l'invention, le procédé de régénération d'une solution de MEG contenant de l'eau et des sels dissous comporte, et peut consister en, les étapes suivantes, de préférence dans cet ordre :
a) une vaporisation sous vide de la solution de MEG contenant de l'eau et des sels dissous 100 dans une première unité de vaporisation sous vide 1000. Cette vaporisation sous vide permet de produire un effluent gazeux comportant du MEG et de l'eau 101, et un résidu liquide enrichi en MEG et en sels 104. Une première partie 106 du résidu liquide enrichi en MEG et en sels 104 est recyclée dans la première unité de vaporisation sous vide 1000.
b) De manière préférée, et tel qu'illustré à la figure 2, on envoie une deuxième partie 105 du résidu liquide enrichi en MEG et en sels 104 issue de l'étape a) dans un réservoir 1002, dans lequel on baisse la température du flux 105 de manière à précipiter des sels inorganiques. On forme alors un flux enrichi en sels précipités 108, dont une fraction 109 est de préférence recyclée dans le réservoir 1002, et un flux appauvri en sels précipités 107 recyclé dans l'unité de vaporisation sous vide 1000 à l'étape a).
c) On envoie, de préférence de manière intermittente, une deuxième partie 105 dudit résidu liquide enrichi en MEG et en sels 104 issue de l'étape a) tel qu'illustré à la figure 1 (cas sans étape b)), ou au moins une fraction 110 dudit flux enrichi en sels précipités 108 obtenu à l'étape b) tel qu'illustré à la figure 2, dans une zone de séparation solide/liquide 1003 pour séparer un flux contenant des sels précipités comportant des sels inorganiques 111 et un effluent liquide comportant des sels dissous parmi lesquels des sels d'acides carboxyliques 112.
d) On envoie, de préférence de manière intermittente, une partie 114 de l'effluent liquide 112 obtenu à l'étape c) dans une unité de séparation 1004 différente de la première unité de vaporisation sous vide 1000, pour opérer une séparation entre le MEG et au moins en partie les sels d'acides carboxyliques ou des acides carboxyliques susceptibles de produire lesdits sels d'acides carboxyliques du MEG. On forme alors un flux de MEG appauvri en sels d'acides carboxyliques ou en acides carboxyliques 115 et un flux résiduel enrichi en sels d'acides carboxyliques ou en acides carboxyliques 116.
e) On recycle le flux de MEG appauvri en sels d'acides carboxyliques ou en acides carboxyliques 115 à l'étape a) ou on mélange le flux de MEG appauvri en sels d'acides carboxyliques ou en acides carboxyliques 115 et l'effluent gazeux comportant du MEG et de l'eau 101 issu de l'étape a), et on utilise ledit effluent gazeux comportant du MEG et de l'eau 101 issu de l'étape a) ou ledit mélange pour produire la solution régénérée de MEG.

Avantageusement, le procédé de régénération selon l'invention comporte en outre une ou plusieurs
- une étape de purification de la solution de MEG riche 100, contenant de l'eau et des sels dissous, préalablement à l'étape a), en particulier une vaporisation à pression sensiblement atmosphérique, pour former un flux d'eau (effluent gazeux enrichi en eau) et une solution aqueuse de MEG appauvrie en eau et contenant des sels, envoyée en totalité à l'étape a) (non représenté dans les figures);
- et/ou une étape f) de purification de l'effluent gazeux 101 issu de l'étape a) dans une unité de purification 1001 pour former un flux d'eau 103 et une solution régénérée de MEG 102, comportant de préférence de 70% à 95% poids de MEG.

L'étape f) est de préférence une distillation sous vide, avantageusement opérée à une pression comprise entre 0,01 MPa et 0,07 MPa.

### Solution de MEG riche 100 à régénérer

La solution aqueuse de MEG 100 à régénérer contient des sels dissous, potentiellement jusqu'à plusieurs pourcents molaires, qui comprennent des sels inorganiques, comme le chlorure de sodium, chlorure de potassium, chlorure de calcium, bicarbonate de sodium, etc., mais qui peuvent aussi comprendre des sels organiques, tels que des sels d'acides carboxyliques, par exemple de type formate (HCOO⁻), l'acétate (CH₃COO⁻), le propionate (C₂H₅COO⁻), le butyrate (C₃H₇COO⁻), etc.

Les sels d'acides carboxyliques sont généralement présents dans la solution aqueuse de MEG 100 à régénérer, qui entre dans la première unité de vaporisation sous vide 1000 à l'étape a), et peuvent provenir du gaz naturel et/ou de l'eau de la formation. Ainsi le procédé selon l'invention vise notamment à éliminer au moins en partie, et de préférence en totalité, les sels d'acides carboxyliques présents dans la solution de MEG riche envoyée à l'étape de vaporisation sous vide a), et qui s'accumulent lors du procédé de régénération.

Dans la présente description, la solution aqueuse de MEG 100 à régénérer qui contient des sels dissous est aussi appelée solution de MEG « riche ».

La solution de MEG 100 riche contient généralement entre 20 % poids et 50 % poids de MEG. Le complément de la solution est de l'eau, aux impuretés près.

La solution de MEG riche peut aussi contenir d'autres impuretés, généralement en faible quantité, de quelques dizaines de ppm molaires à quelques pourcents molaires, par exemple des hydrocarbures issus notamment de formations souterraines, des gaz dissous tel que du CO₂, del'H₂S.

Les sels inorganiques peuvent précipiter dans les conditions opératoires de certaines étapes du procédé, en particulier à l'étape a) de vaporisation, mais également en particulier à l'étape b) quand cette étape est mise en oeuvre. Ils sont séparés du reste de la solution d'eau et de MEG à l'étape c) de séparation solide/liquide, et évacués hors du procédé et de l'installation de régénération.

Les sels d'acides carboxyliques, qui pour l'essentiel ne précipitent pas dans les conditions opératoires des étapes du procédé, sont principalement séparés du reste de la solution d'eau et de MEG et sont évacués hors du procédé et de l'installation à l'étape d) de séparation.

Le pH de la solution de MEG 100 à régénérer est de préférence compris entre 7 et 14, typiquement de l'ordre de 10,5 à 11. Un tel pH peut être obtenu par ajustement du pH, par exemple par ajout de soude dans la solution de MEG riche préalablement à son envoi dans l'unité de vaporisation sous vide 1000 à l'étape a).

Une tel pH empêche les sels d'acides carboxyliques d'être sous la forme d'acides carboxyliques, et les empêche d'être vaporisés avec l'eau et le MEG lors de l'étape de vaporisation a).

Les différentes étapes du procédé selon l'invention sont détaillées ci-après.

### Etape a) de vaporisation sous vide

Lors de cette étape a), la solution de MEG riche 100 est envoyée à l'unité de vaporisation sous vide 1000 pour y être partiellement vaporisée.

L'unité de vaporisation sous vide 1000 peut être tout dispositif de vaporisation pouvant être opéré sous vide connu de l'homme du métier, par exemple un ballon, aussi parfois appelé ballon de détente (ou « flash » en anglais), du fait de la vaporisation produite par le vide opéré dans le ballon et/ou en présence d'un système de chauffage. Ce peut aussi être une colonne de distillation sous vide.

Cette vaporisation sous vide produit un effluent gazeux comportant, et de préférence constitué par, du MEG et de l'eau 101, et un résidu liquide enrichi en MEG et en sels 104. En effet, la vaporisation permet de séparer un mélange MEG/eau composant essentiellement l'effluent gazeux 101, du reste de la solution de MEG riche 100 entrant dans l'unité 1000, éventuellement en mélange avec un ou plusieurs flux de recycle (107, 113, 115) du procédé décrits plus loin. Du fait de la vaporisation, les sels, qu'ils soient inorganiques ou organiques, se concentrent dans la phase liquide très riche en MEG (comportant typiquement entre 50 % poids et 99 % poids de MEG, de préférence entre 70 % poids et 99 % poids de MEG, par exemple entre 85 % poids et 99 % poids de MEG) contenue dans l'unité de vaporisation sous vide 1000, et extraite de l'unité 1000 sous la forme du résidu liquide enrichi en MEG et en sels 104. Les impuretés les plus volatiles (CO₂ et hydrocarbures dissous) potentiellement présents dans la solution de MEG riche 100 sont également évaporés dans l'effluent gazeux 101.

L'effluent gazeux 101 contient de préférence sensiblement les mêmes proportions de MEG et d'eau que la solution de MEG 100 à régénérer entrant dans l'unité 1000, du fait des flux de recycle (107, 113, 115) dans l'unité 1000. En particulier, l'effluent gazeux 101 comporte de préférence entre 20 % poids et 50 % poids de MEG si la solution de MEG 100 à régénérer comporte entre 20 % poids et 50 % poids de MEG. Le complément au MEG dans l'effluent gazeux 101 étant de l'eau, aux impuretés près.

Une première partie 106 du résidu liquide enrichi en MEG et en sels 104 est recyclée dans la première unité de vaporisation sous vide 1000.

La concentration en sels dans le résidu liquide 104 est de préférence comprise entre 7% poids et 30% poids (du résidu liquide), tous types de sels confondus, et plus préférentiellement comprise entre 9 % poids et 12% poids (du résidu liquide).

La concentration en sels inorganiques dans la boucle de recycle interne formée par le flux 106 est de préférence comprise entre 5% poids et 20% poids, de préférence entre 9% poids et 12 % poids. Cette concentration est de préférence maintenue dans cette gamme par le flux 105 provenant de l'unité 1000.

La concentration en sels organiques est de préférence inférieure à 30 % poids du résidu liquide, plus préférentiellement inférieure à 10 % poids du résidu liquide, encore plus préférentiellement inférieure à 3 % poids du résidu liquide, et de manière encore plus préférée inférieure à 1 % poids du résidu liquide.

Une deuxième partie du résidu liquide enrichi en MEG et en sels 104 est envoyée soit directement à l'étape c) de séparation liquide/solide, comme illustré à la figure 1, soit à une étape b) de concentration et précipitation des sels sous la forme du flux 105 comme illustré à la figure 2.

Le recyclage de la première partie 106 du résidu liquide enrichi en MEG et en sels 104 dans l'unité 1000 crée une boucle de recirculation, aussi appelée boucle de recycle interne, qui permet notamment de contrôler le taux de sels dans le résidu liquide enrichi en MEG et en sels 104 via l'envoi d'une deuxième partie soit à l'étape c) (flux 105 de la figure 1), soit à l'étape b) (flux 105 de la figure 2).

De préférence, la deuxième partie 105 du résidu liquide enrichi en MEG et en sels 104, envoyée soit directement à l'étape c) soit à l'étape b), constitue entre 3 et 30 % poids du résidu liquide enrichi en MEG et en sels 104, et plus généralement entre 8 % poids et 12 % poids du résidu 104. Par exemple la deuxième partie 105 du résidu liquide enrichi en MEG et en sels 104, envoyée à l'étape b), représente 10 % poids du résidu liquide enrichi en MEG et en sels 104.

L'étape a) est de préférence opérée à une température comprise entre 100°C et 155°C, et à une pression comprise entre 0,01 MPa et 0,07 MPa.

La pression d'opération dans l'unité 1000 peut être obtenue grâce à une pompe à vide, typiquement placée en aval de l'unité 1000, au sens de la circulation des effluents sortant de l'unité 1000, par exemple en aval de l'effluent gazeux 101, ou encore en aval du flux d'eau 103 (vapeur d'eau) issu d'une colonne de distillation 1001 recevant l'effluent gazeux comportant de l'eau et du MEG 101 lorsqu'une telle colonne est mise en oeuvre. La température opératoire peut être obtenue grâce à un apport de chaleur par la première partie 106 du résidu liquide enrichi en MEG et en sels 104 recyclée dans l'unité 1000, qui est préalablement passée dans un échangeur de chaleur (non représenté) afin d'être chauffée.

L'effluent gazeux comportant du MEG et de l'eau 101 est utilisé pour produire la solution de MEG régénérée, soit directement, notamment quand une étape de purification de la solution de MEG riche préalable à l'étape a) est mise en oeuvre (i.e. étape de distillation atmosphérique), soit indirectement, notamment après une étape supplémentaire de purification f) dudit effluent gazeux 101 décrite plus loin, où il est envoyé, seul ou en mélange avec le flux 115 issu de l'étape d), et de préférence seul.

### Etape b) de précipitation de sels inorganiques - optionnelle

Le procédé opère de préférence avec l'étape b), comme illustré à la figure 2.

A l'étape b), on envoie une deuxième partie 105 du résidu liquide enrichi en MEG et en sels 104 issue de l'étape a) dans un réservoir 1002, dans lequel on baisse la température du flux 105 de manière à précipiter des sels inorganiques.

De préférence, l'envoi de la deuxième partie 105 du résidu liquide enrichi en MEG et en sels 104 dans le réservoir 1002 est réalisé en continu ou de manière intermittente, et de manière préférée en continu.

Ce stockage d'une partie 105 du résidu liquide 104 dans un réservoir 1002 et sa baisse de température provoque et/ou accroît la précipitation des sels inorganiques.

On forme alors un flux enrichi en sels précipités 108 et un flux appauvri en sels précipités 107 recyclé dans l'unité de vaporisation sous vide 1000 à l'étape a).

Une première fraction 109 du flux enrichi en sels précipités 108 est de préférence recyclée dans le réservoir 1002. Une deuxième fraction 110 du flux enrichi en sels précipités 108, complémentaire à la première fraction 109, est envoyée à l'étape c). La fraction envoyée à l'étape c) dépend du système de séparation solide/liquide, et peut être intermittente ou continue.

La deuxième fraction 110 du flux enrichi en sels précipités 108 envoyée à l'étape c) peut constituer jusqu'à la totalité du flux enrichi en sels précipités 108 pour un fonctionnement intermittent. Toutefois lorsque le système de séparation solide/liquide employé à l'étape c) opère avec un fonctionnement intermittent, par exemple un système de séparation solide/liquide de type filtration par centrifugeuse à panier, on peut évaluer que moins de 1% du flux journalier circulant 108 est envoyé vers 1003.

Le réservoir 1002 constitue une zone de séparation liquide/solide « passive », par décantation : les sels précipités en suspension s'accumulent dans le fond de manière gravitaire, alors que le liquide remonte. C'est ce dernier qui va former le flux appauvri en sels précipités 107 recyclé dans l'unité de vaporisation sous vide 1000 à l'étape a).

De préférence, la pression dans le réservoir 1002 est de l'ordre de 0,1 MPa, par exemple comprise entre 0,05 MPa et 0,15 MPa, plus préférentiellement comprise entre 0,08 MPa et 0,13 MPa, et la température comprise entre 40°C et 155°C, de préférence entre 55°C et 70°C. Un gradient de température, pression, concentration en sels peut s'établir dans le réservoir 1002.

De préférence, on envoie la deuxième fraction 110 du flux enrichi en sels précipités 108 dans l'unité de séparation solide/liquide 1003 de manière intermittente.

Cet envoi intermittent est avantageusement effectué si le concentration en sels inorganiques du flux enrichi en sels précipités 108 est comprise entre 10% poids et 50% poids, de préférence entre 15% poids et 30% poids.

L'envoi intermittent de la fraction 110 dans l'unité de séparation solide/liquide peut par exemple être déclenché au moyen du contrôle d'un paramètre lié à la concentration en sels inorganiques du flux 108, par exemple la densité. On détermine par exemple une valeur seuil de densité correspondant à une valeur de concentration en sels inorganiques minimale, par exemple de 10%, ou de préférence de 15%, ou encore une gamme de valeurs seuils de densité correspondant aux gammes de concentration en sels inorganiques mentionnées ci-dessus, et on déclenche l'envoi de la fraction 110 dans l'unité 1003 lorsque la densité mesurée, par exemple par un densimètre, est au-delà de cette valeur seuil ou dans la gamme de valeurs seuils.

Un fonctionnement non intermittent, i.e. continu, pour l'envoi de la deuxième fraction 110 du flux enrichi en sels précipités 108 à l'étape c) est possible: la fraction 110 du flux enrichi en sels précipités 108 peut être envoyée en continu dans l'unité de séparation liquide/solide 1003 à l'étape c).

### Etape c) de séparation liquide-solide pour l'élimination des sels inorganiques précipités

Lors de cette étape c), on envoie, de préférence de manière intermittente, la deuxième partie 105 du résidu liquide enrichi en MEG et en sels 104 issu de l'étape a), quand le procédé ne comporte pas l'étape b) comme illustré à la figure 1, dans une zone de séparation solide/liquide 1003 pour séparer un flux contenant des sels précipités comportant des sels inorganiques 111 et un effluent liquide comportant encore des sels dissous parmi lesquels des sels d'acides carboxyliques 112.

Alternativement, quand le procédé comporte l'étape b) comme illustré à la figure 2, on envoie, de préférence de manière intermittente, la deuxième fraction 110 du flux enrichi en sels précipités 108 obtenu à l'étape b) dans la zone de séparation solide/liquide 1003 pour séparer un flux contenant des sels précipités comportant des sels inorganiques 111 et un effluent liquide comportant encore des sels dissous parmi lesquels des sels d'acides carboxyliques 112.

Cette étape permet la séparation des sels précipités, principalement des sels inorganiques, du reste du liquide reçu dans l'unité 1003. Cette séparation solide/liquide est réalisée par tout moyen connu de l'homme du métier, par exemple un dispositif de filtration ou une centrifugeuse, et de préférence une centrifugeuse.

Le flux contenant les sels précipités 111 comprend un gâteau de sels exclusivement composé de sels inorganiques, ainsi qu'un peu de MEG et d'eau et de sels dissous (inorganiques et organiques à saturation). La concentration en sels inorganiques du flux 111 est par exemple comprise entre 80 % poids et 100 % poids, de préférence comprise entre 85 % poids et 98 % poids, et plus préférentiellement comprise entre 90% poids et 95% poids.

Le flux liquide 112 sortant de l'unité de séparation solide/liquide 1003, e.g. une centrifugeuse, comprend du MEG, de l'eau, des sels inorganiques dissous à saturation, et des sels d'acides carboxyliques dissous.

Une portion 114 ou la totalité de l'effluent liquide 112 est envoyée à l'étape de séparation d) pour l'éliminations des sels d'acides carboxyliques décrite plus loin. Si seule une portion 114 de l'effluent liquide 112 est envoyée à l'étape de séparation d), l'autre portion 113 de l'effluent liquide 112 est à nouveau envoyée dans l'unité 1000 à l'étape a) de vaporisation sous vide.

De préférence, l'étape c) est opérée à une température comprise entre 50°C et 90°C et à pression sensiblement atmosphérique. Par exemple la pression est comprise entre 0,1 MPa et 0,15 MPa, de préférence entre 0,1 MPa et 0,12 MPa.

Comme déjà mentionné plus haut, pour la figure 2, l'envoi de la deuxième fraction 110 du flux enrichi en sels précipités 108 dans l'unité de séparation liquide/solide 1003 est de préférence effectué de manière intermittente.

Pour le mode de réalisation illustré à la figure 1, qui ne comporte pas d'étape b), un envoi intermittent de la deuxième partie 105 du flux 104, issu de l'unité de vaporisation sous vide 1000, dans l'unité de séparation liquide solide 1003 est également possible.

Cependant un fonctionnement continu est possible, dans lequel l'unité de séparation liquide/solide 1003 reçoit en continu un flux liquide duquel les sels cristallisés sont séparés du reste du flux liquide, que ce flux liquide soit la première fraction 110 du flux 108 arrivant depuis le réservoir 1002 (cas du mode de réalisation illustré à la figure 2) ou la deuxième partie 105 du résidu liquide enrichi en MEG et en sels 104 issu de l'unité de vaporisation sous vide 1000 à l'étape a) (cas du mode de réalisation illustré à la figure 1).

Le choix d'un fonctionnement intermittent ou continu pour l'envoi du flux liquide dans l'unité de séparation solide liquide 1003 peut dépendre de la technologie utilisée pour la séparation solide liquide, par exemple le type de système de filtration ou centrifugeuse utilisé.

### Etape d) de séparation pour l'éliminations des sels d'acides carboxyliques ou des acides carboxyliques

Selon l'invention, à l'étape d), on envoie une portion 114 ou la totalité de l'effluent liquide 112 obtenu à l'étape c) dans une unité de séparation 1004 différente de la première unité de vaporisation sous vide 1000 pour séparer au moins en partie les sels d'acides carboxyliques, ou les acides carboxyliques susceptibles de produire lesdits sels d'acides carboxyliques, du MEG, et former un flux de MEG appauvri en sels d'acides carboxyliques ou en acides carboxyliques 115 et un flux résiduel enrichi en sels d'acides carboxyliques ou en acides carboxyliques 116. L'unité de séparation 1004 est également différente de l'unité de séparation liquide/solide 1003 de l'étape c). En effet, elle vise à séparer des composés différents. L'unité de séparation 1004 est distincte physiquement de l'unité de vaporisation sous vide 1000 et de l'unité de séparation liquide/solide 1003.

La mise en oeuvre de l'étape d) dans le procédé selon l'invention permet de traiter un flux de MEG 114 ou 112 d'un débit réduit, comparé au flux 101, ce qui permet de réduire la taille des équipements.

A l'étape d), par séparation « au moins en partie » des sels d'acides carboxyliques, ou des acides carboxyliques susceptibles de produire lesdits sels, du MEG, on entend :
- soit qu'une seule partie de la quantité totale des sels d'acides carboxyliques est séparée du MEG, tout type de sels d'acide carboxyliques confondus, étant entendu que la totalité est de préférence séparée (« au moins »),
- soit qu'une seule sous-famille d'acides carboxyliques susceptibles de produire certains sels carboxyliques est préférentiellement séparée du MEG. Ce dernier cas dépend des performances des techniques de séparation et s'applique notamment au(x) mode(s) de réalisation où l'étape d) est réalisée par acidification de la solution de MEG et stripage de la solution acidifiée comme détaillé plus loin.

De manière préférée, l'envoi d'une portion 114 ou la totalité de l'effluent liquide 112 obtenu à l'étape c) dans l'unité de séparation 1004 est effectué de manière intermittente.

De préférence, la totalité de l'effluent liquide 112 obtenu à l'étape c) est envoyé de manière intermittente à l'unité de séparation 1004.

Le flux liquide 112 qui sort de l'unité de séparation solide/liquide 1003 comprend du MEG, de l'eau, des sels inorganiques dissous à saturation, et des sels d'acides carboxyliques dissous. La teneur en sels d'acides carboxyliques dissous croit au fur et à mesure du temps jusqu'à attendre une valeur seuil.

Avantageusement, cet envoi intermittent est effectué si un paramètre lié à la concentration en sels d'acides carboxyliques dans l'effluent liquide 112, tel que la viscosité dynamique, est supérieur à une valeur seuil déterminée. Par exemple, l'envoi de la portion 114 ou la totalité de l'effluent liquide 112 dans l'unité de séparation 1004 peut être déclenché si la viscosité dynamique est supérieure à une valeur seuil correspondant à 3 % poids de sels d'acides carboxyliques dans l'effluent 112, plus préférentiellement 1% en poids de sels d'acides carboxyliques. De préférence, le déclenchement de l'envoi de la portion 114 ou la totalité de l'effluent liquide 112 dans l'unité de séparation 1004 est effectué pour une concentration en sels d'acides carboxyliques dans l'effluent liquide 112 comprise entre 1 % poids et 30 % poids, de préférence entre 1 % poids et 10 % poids. Une marge peut être prise en compte pour déterminer la valeur seuil de viscosité. Ladite valeur seuil de viscosité, avec la marge, est une valeur sélectionnée pour éviter que l'effluent liquide 112 devienne difficile à opérer. Lorsque la viscosité de l'effluent liquide atteint la valeur seuil de viscosité, ce qui signifie que le procédé est toujours opérable du fait de la marge sur la valeur seuil, on envoie la portion 114 ou la totalité de l'effluent liquide 112 dans l'unité de séparation 1004. Si la concentration requise n'est pas atteinte, en particulier si la valeur seuil de viscosité n'est pas atteinte, dans l'effluent liquide 112, le flux 114 est nul et l'intégralité de l'effluent liquide 112 peut être recyclée vers la première unité de vaporisation sous vide 1000, afin d'augmenter progressivement la concentration des sels d'acides carboxyliques dans l'effluent liquide 112.

Tout moyen connu de l'homme du métier peut être utilisé pour évaluer la quantité de sels d'acides carboxyliques. Par exemple la mesure de la viscosité dynamique de l'effluent liquide 112, tel que des viscosimètres de type vibrant ou à fréquence de résonnance.

Selon une variante du fonctionnement intermittent, une portion 114 de l'effluent liquide 112 obtenu à l'étape c) est envoyé de manière intermittente dans l'unité de séparation 1004, alors qu'une autre portion 113 est recyclée dans l'unité 1000 à l'étape a) de vaporisation sous vide pendant le même temps où la portion 114 est envoyée à l'étape d).

Cependant un fonctionnement en continu est possible, dans lequel l'unité de séparation 1004 reçoit en continu une portion 114 de l'effluent liquide 112. En pratique, dans le cas d'une mise en oeuvre en continu, un débit prédéterminé est établi pour la portion 114 de l'effluent liquide 112 à envoyer à l'unité de séparation 1004.

Alors que l'étape c) de séparation solide/liquide vise à retirer du flux de MEG les sels d'acides inorganiques précipités, l'étape d) vise à éliminer les sels d'acides carboxyliques dissous, ou certains acides carboxyliques susceptibles de former des sels d'acides carboxyliques, dudit flux 114.

Pour ce faire, plusieurs manières d'opérer à cette étape d) sont possibles.

A l'étape d), l'unité de séparation 1004 est une deuxième unité de vaporisation différente de la première unité de vaporisation.

D'autres technologies permettant d'opérer la séparation entre les sels d'acides carboxyliques (ou les acides carboxyliques susceptibles de produire lesdits sels d'acides carboxyliques) et le MEG peuvent être utilisées sans faire partie du cadre de la présente invention, par exemple la mise en oeuvre de résines échangeuses d'ions.

Selon l'invention, l'unité de séparation 1004 est une deuxième unité de vaporisation sous vide, distincte de la première unité de vaporisation sous vide 1000. On vaporise sous vide la portion 114 de l'effluent liquide 112 obtenu à l'étape c) pour former une vapeur enrichie en MEG et appauvrie en sels d'acides carboxyliques 115, et un effluent résiduel liquide enrichi en sels d'acides carboxyliques 116.

Selon cette mise en oeuvre, il est possible d'envoyer un flux supplémentaire 117 dans la deuxième unité de vaporisation pour augmenter la quantité de MEG récupérée dans le flux 115. De préférence ledit flux supplémentaire 117 est de l'eau : on envoie un flux d'eau dans la deuxième unité de vaporisation, ce qui permet notamment d'abaisser la température opératoire, favorisant alors la vaporisation du MEG et de l'eau et ainsi la séparation entre les sels d'acides carboxyliques et le MEG. Ce flux supplémentaire 117 peut être également de la vapeur d'eau ou un gaz inerte comme l'azote ou tout autre composé ou mélange de composés améliorant la récupération du MEG. Selon cette mise en oeuvre, l'étape d) est de préférence opérée à une température comprise entre 120°C et 155°C, et une pression comprise entre 0,001 MPa et 0,05 MPa.

Selon cette mise en oeuvre, la vaporisation sous vide à l'étape d) permet de séparer en totalité les sels d'acides carboxyliques du MEG récupéré avec le flux 115.

Selon une autre mise en oeuvre, l'unité de séparation 1004 est une unité d'extraction liquide/liquide,

### Etape e) de récupération du flux de MEG issu de l'étape d) et production de la solution régénérée

A cette étape e), soit on recycle le flux de MEG appauvri en sels d'acides carboxyliques (ou en acides carboxyliques) 115 dans la première unité de vaporisation sous vide 1000 à l'étape a), soit on mélange ledit flux de MEG 115 avec l'effluent gazeux comportant du MEG et de l'eau 101 issu de l'étape a) (non représenté dans les figures), et on utilise ledit effluent gazeux comportant du MEG et de l'eau 101 issu de l'étape a) ou ledit mélange pour produire la solution régénérée de MEG.

Le choix entre ces deux options (recycle à l'étape a) ou mélange avec l'effluent 101) dépend de la composition en impuretés du flux de MEG 115, qui lui-même dépend du type de séparation utilisée à l'étape d).

De manière préférée, on recycle le flux de MEG appauvri en sels d'acides carboxyliques (ou en acides carboxyliques) 115 dans la première unité de vaporisation sous vide 1000 à l'étape a). Dans cette configuration, les sels inorganiques que le flux peut contenir sous forme dissoute à saturation peuvent être à nouveau éliminés à l'étape a).

La production de la solution régénérée de MEG peut en effet être obtenue indirectement à partir de l'effluent gazeux comportant du MEG et de l'eau 101 issu de l'étape a) ou dudit mélange, soit en mettant en oeuvre l'étape f) de purification telle que décrite plus bas (comme représenté aux figures 1 et 2), soit en mettant en oeuvre l'étape f) de purification et une étape l'étape de vaporisation à pression atmosphérique de la solution de MEG riche à régénérer préalablement à l'étape a) également décrite plus bas dans la partie relative à l'étape f).

La production de la solution régénérée de MEG peut également être obtenue directement (non représenté dans les figures) à partir de l'effluent gazeux comportant du MEG et de l'eau 101 issu de l'étape a) ou dudit mélange, en mettant en oeuvre l'étape de vaporisation à pression atmosphérique de la solution de MEG riche à régénérer préalablement à l'étape a), décrite plus bas dans la partie relative à l'étape f).

La solution régénérée de MEG comporte de préférence entre 70 % poids et 95 % poids de MEG, voire entre 70 % poids et 90% poids de MEG.

Cette solution régénérée de MEG ne contient pas de sels ou éventuellement des sels résiduels dans un teneur compatible avec les spécifications requises pour sa réutilisation en tant qu'inhibiteur d'hydrates, typiquement inférieure à 100 ppm poids.

Cette solution peut alors être à nouveau utilisée en tant qu'inhibiteur de formation d'hydrates, pour la production et le transport de gaz naturel par exemple.

### Etape f) de purification de l'effluent gazeux issu de l'étape a) - optionnelle

Cette étape f) est optionnelle.

Le procédé selon l'invention opère de préférence avec cette étape f).

Le procédé peut ainsi comprendre en outre une étape f) de purification de l'effluent gazeux 101 issu de l'étape a) dans une unité de purification 1001 pour former un flux d'eau 103 et la solution régénérée de MEG 102, comportant de préférence de 70 % à 95 % poids de MEG. Cette solution régénérée de MEG remplie les spécifications requises pour sa réutilisation en tant qu'inhibiteur d'hydrates.

Le flux d'eau 103 peut être récupéré dans un ballon de reflux, où une séparation eau / hydrocarbures peut être effectuée si nécessaire.

De préférence l'étape de purifications f) est une distillation sous vide dans une colonne de distillation sous vide, avantageusement opérée à une pression inférieure à 0,1 MPa, et de préférence comprise entre 0,01 MPa et 0,07 MPa.

Selon un mode de réalisation, l'unité de purification 1001 peut recevoir , à la place du seul effluent gazeux 101, un mélange du flux de MEG 115 issu de l'étape de séparation d) avec l'effluent gazeux 101 issu de l'étape a) si la composition du flux 115 permet un tel mélange, afin de produire la solution de MEG régénérée 102.

Selon un mode de réalisation, quand l'étape f) n'est pas réalisée, on réalise une étape de vaporisation à pression sensiblement atmosphérique de la solution de MEG riche à régénérer préalablement à l'étape a), dans une unité de vaporisation initiale, de préférence à une température comprise entre 100°C et 140°C et une pression comprise entre 0,1 MPa et 0,15 MPa , de préférence entre 0,1 MPa et 0,12 MPa, pour produire un effluent gazeux enrichi en eau et un résidu liquide enrichi en MEG et en sels dont la totalité est envoyée à l'étape a).

Selon un mode de réalisation, le procédé comporte à la fois ladite étape de vaporisation à pression sensiblement atmosphérique de la solution de MEG riche à régénérer préalablement à l'étape a), et l'étape f) de purification de l'effluent gazeux issu de l'étape a).

Selon une mise en oeuvre préférée de l'invention, le procédé comporte les étapes suivantes :
a) on vaporise sous vide la solution 100 dans la première unité de vaporisation sous vide 1000 pour produire l'effluent gazeux comportant du MEG et de l'eau 101, et le résidu liquide enrichi en MEG et en sels 104 dont une première partie 106 est recyclée dans la première unité de vaporisation sous vide 1000 ;
b) on envoie une deuxième partie 105 dudit résidu liquide enrichi en MEG et en sels 104 dans le réservoir 1002 dans lequel on baisse la température de ladite deuxième partie 105 de manière à précipiter des sels inorganiques pour former le flux enrichi en sels précipités 108 dont une première fraction 109 est de préférence recyclée dans le réservoir 1002 et le flux appauvri en sels précipités 107 recyclé dans l'unité de vaporisation sous vide 1000 à l'étape a) ;
c) on envoie, de préférence de manière intermittente, une deuxième fraction 110 dudit flux enrichi en sels précipités 108 obtenu à l'étape b) dans la zone de séparation solide/liquide 1003, comportant de préférence une centrifugeuse, pour séparer le flux contenant des sels précipités comportant des sels inorganiques 111 et l'effluent liquide comportant des sels dissous parmi lesquels des sels d'acides carboxyliques 112;
d) on envoie de manière intermittente la totalité dudit effluent liquide 112 obtenu à l'étape c) dans une deuxième unité de vaporisation 1004, différente de la première unité de vaporisation 1000, ladite deuxième unité de vaporisation 1004 étant opérée de préférence à une température comprise entre 120°C et 155°C, et une pression comprise entre 0,01 MPa et 0,07 MPa, et on vaporise ledit effluent liquide 112 pour former une vapeur enrichie en MEG et appauvrie en sels d'acides carboxyliques 115 et un effluent résiduel liquide enrichi en sels d'acides carboxyliques 116;
e) on recycle ladite vapeur enrichie en MEG et appauvrie en sels d'acides carboxyliques 115 à l'étape a), et on utilise ledit effluent gazeux comportant du MEG et de l'eau 101 issu de l'étape a) pour produire la solution régénérée de MEG.

Selon cette mise en oeuvre préférée, on effectue une étape de purification f) dans une unité de purification 1001 de l'effluent gazeux comportant du MEG et de l'eau 101, pour former un flux de vapeur d'eau 103 et la solution régénérée de MEG 102, comportant de préférence de 70 % à 95 % poids de MEG, ladite étape de purification étant une distillation sous vide, de préférence opérée à une pression comprise entre 0,01 MPa et 0,07 MPa. Le procédé selon l'invention, par l'enchainement des étapes décrites, permet ainsi avantageusement de récupérer un maximum de MEG lors de la régénération de la solution de MEG riche, tout en éliminant les sels inorganiques et au moins en partie les sels d'acides carboxyliques présents dans la solution.

## Revendications

1. Procédé de régénération d'une solution de monoéthylène glycol (MEG) contenant de l'eau et des sels dissous, comportant les étapes suivantes :
a) on vaporise sous vide ladite solution (100) dans une première unité de vaporisation (1000) pour produire un effluent gazeux comportant du MEG et de l'eau (101), et un résidu liquide enrichi en MEG et en sels (104) dont une première partie (106) est recyclée dans ladite première unité de vaporisation sous vide (1000) ;
b) éventuellement on envoie une deuxième partie (105) dudit résidu liquide enrichi en MEG et en sels dans un réservoir (1002) dans lequel on baisse la température de ladite deuxième partie (105) dudit résidu liquide enrichi en sels de manière à précipiter des sels inorganiques pour former un flux enrichi en sels précipités (108) dont une première fraction (109) est de préférence recyclée dans le réservoir (1002) et un flux appauvri en sels précipités (107) recyclé dans l'unité de vaporisation sous vide (1000) à l'étape a) ;
c) on envoie, de préférence de manière intermittente, ladite deuxième partie (105) dudit résidu liquide enrichi en MEG et en sels (104) issue de l'étape a) ou une deuxième fraction (110) dudit flux enrichi en sels précipités (108) obtenu à l'étape b), dans une zone de séparation solide/liquide (1003) pour séparer un flux contenant des sels précipités comportant des sels inorganiques (111) et un effluent liquide comportant des sels dissous parmi lesquels des sels d'acides carboxyliques (112);
d) on envoie, de préférence de manière intermittente, une portion (114) ou la totalité dudit effluent liquide (112) obtenu à l'étape c) dans une deuxième unité de vaporisation (1004) différente de la première unité de vaporisation sous vide (1000) pour opérer une séparation entre le MEG et au moins en partie les sels d'acides carboxyliques en vaporisant sous vide la portion (114) ou la totalité dudit effluent liquide (112) obtenu à l'étape c) pour former une vapeur enrichie en MEG et appauvrie en sels d'acides carboxyliques (115) et un effluent résiduel liquide enrichi en sels d'acides carboxyliques (116);
e) on recycle ladite la vapeur enrichie en MEG et appauvrie en sels d'acides carboxyliques (115) à l'étape a), ou on mélange ladite la vapeur enrichie en MEG et appauvrie en sels d'acides carboxyliques (115) avec l'effluent gazeux comportant du MEG et de l'eau (101) issu de l'étape a), et on utilise ledit effluent gazeux comportant du MEG et de l'eau (101) issu de l'étape a) ou ledit mélange pour produire la solution régénérée de MEG.

2. Procédé selon la revendication 1, dans lequel à l'étape e) la vapeur enrichie en MEG et appauvrie en sels d'acides carboxyliques (115) issu de l'étape d) est recyclé à l'étape a).

3. Procédé selon la revendication 2, dans lequel on effectue en outre une étape f) de purification de l'effluent gazeux (101) issu de l'étape a) dans une unité de purification (1001) pour former un flux d'eau (103) et la solution régénérée de MEG (102), comportant de préférence de 70% à 95% poids de MEG.

4. Procédé selon la revendication 3, dans lequel l'étape de purifications f) est une distillation sous vide, de préférence opérée à une pression comprise entre 0,01 MPa et 0,07 MPa.

5. Procédé selon l'une quelconque des revendications précédentes, dans lequel, préalablement à l'étape a), on réalise une vaporisation à pression atmosphérique de la solution de MEG contenant de l'eau et des sels dissous (100) à régénérer dans une unité de vaporisation initiale, de préférence à une température comprise entre 100°C et 155°C et une pression comprise entre 0,1 MPa et 0,15 MPa, pour produire un effluent gazeux enrichi en eau et un résidu liquide enrichi en MEG et en sels dont la totalité est envoyée à l'étape a).

6. Procédé selon l'une quelconque des revendications précédentes, dans lequel l'étape a) est opérée à une température comprise entre 120°C et 155°C, et une pression comprise entre 0,01 MPa et 0,07 MPa.

7. Procédé selon l'une quelconque des revendications précédentes, dans lequel l'étape c) est opérée à une température comprise entre 50°C et 90°C et à pression atmosphérique, de préférence à une pression comprise entre 0,1 MPa et 0,15 MPa.

8. Procédé selon l'une quelconque des revendications précédentes, dans lequel on envoie la deuxième fraction (110) dudit flux enrichi en sels précipités (108) issu du réservoir (1002) dans l'unité de séparation solide/liquide (1003) de manière intermittente, de préférence si le concentration en sels inorganiques dudit flux enrichi en sels précipités (108) est comprise entre 10 % poids et 50 % poids, de préférence entre 15 % poids et 30 % poids.

9. Procédé selon l'une des revendications précédentes, dans lequel on envoie de manière intermittente la portion (114) ou la totalité dudit effluent liquide (112) obtenu à l'étape c) dans l'unité de séparation (1004) de l'étape d).

10. Procédé selon l'une des revendications précédentes, dans lequel on envoie un flux supplémentaire liquide ou gazeux (117) dans ladite deuxième unité de vaporisation pour augmenter la quantité de MEG récupérée dans le flux (115) lors de l'étape d), ledit flux étant choisi parmi l'eau, la vapeur d'eau, ou un gaz inerte.

11. Procédé selon l'une des revendications précédentes, dans lequel l'étape d) est opérée à une température comprise entre 120°C et 155°C, et une pression comprise entre 0,01 MPa et 0,07 MPa.

## Patentansprüche

1. Verfahren zur Regenerierung einer Lösung aus Monoethylenglycol (MEG), die Wasser und gelöste Salze enthält, mit folgenden Schritten:
a) Verdampfen der Lösung (100) unter Vakuum in einer ersten Verdampfungseinheit (1000) zum Erzeugen eines Gasaustrags (101), der MEG und Wasser aufweist, sowie eines flüssigen Rests (104), der reich an MEG und an Salzen ist, wovon ein erster Teil (106) in die erste unter Vakuum arbeitende Verdampfungseinheit (1000) zurückgeleitet wird;
b) gegebenenfalls Leiten eines zweiten Teils (105) des flüssigen Rests, der reich an MEG und an Salzen ist, in einen Behälter (1002), in dem die Temperatur des zweiten Teils (105) des flüssigen Rests, der reich an Salzen ist, derart abgesenkt wird, dass anorganische Salze ausgefällt werden und so ein Strom (108), der reich an ausgefällten Salzen ist, wovon ein erster Anteil (109) vorzugsweise in den Behälter (1002) zurückgeleitet wird, und ein Strom (107) entsteht, der weniger ausgefällte Salze aufweist und in die unter Vakuum arbeitende Verdampfungseinheit (1000) in Schritt a) zurückgeleitet wird;
c) Leiten, vorzugsweise mit Unterbrechungen, des zweiten Teils (105) des flüssigen Rests (104), der reich an MEG und an Salzen ist, aus Schritt a) oder eines zweiten Anteils (110) des Stroms (108), der reich an ausgefällten Salzen ist und in Schritt b) erhalten wird, in einen Trennbereich fest/flüssig (1003) zum Abtrennen eines Stroms (111), der ausgefällte Salze enthält, die anorganische Salze umfassen, und eines flüssigen Austrags, der gelöste Salze aufweist, darunter Carbonsäuresalze (112);
d) Leiten, vorzugsweise mit Unterbrechungen, eines Teils (114) des oder des gesamten flüssigen Austrags (112), der in Schritt c) erhalten wird, in eine zweite Verdampfungseinheit (1004), die sich von der ersten unter Vakuum arbeitenden Verdampfungseinheit (1000) unterscheidet, um eine Trennung zwischen dem MEG und zumindest teilweise den Carbonsäuresalzen vorzunehmen, durch Verdampfen des Teils (114) des oder des gesamten flüssigen Austrags (112), der in Schritt c) erhalten wird, unter Vakuum und so Bilden eines Dampfs (115), der reich an MEG ist und weniger Carbonsäuresalze aufweist, und einen flüssigen Restaustrag (116), der reich an Carbonsäuresalzen ist;
e) Zurückleiten des Dampfs (115), der reich an MEG ist und weniger Carbonsäuresalze aufweist, in Schritt a), oder Mischen des Dampfs (115), der reich an MEG ist und weniger Carbonsäuresalze aufweist, mit dem Gasaustrag (101), der MEG und Wasser aufweist, aus Schritt a), und Verwenden des Gasaustrags (101), der MEG und Wasser aufweist, aus Schritt a) oder der Mischung zum Herstellen der regenerierten MEG-Lösung.

2. Verfahren nach Anspruch 1, wobei in Schritt e) der Dampf (115), der reich an MEG ist und weniger Carbonsäuresalze aufweist, aus Schritt d) in Schritt a) zurückgeleitet wird.

3. Verfahren nach Anspruch 2, wobei ferner ein Schritt f) zur Reinigung des Gasaustrags (101) aus Schritt a) in einer Reinigungseinheit (1001) erfolgt und so ein Wasserstrom (103) und die regenerierte MEG-Lösung (102) entsteht, die vorzugsweise 70 Gewichts-% bis 95 Gewichts-% MEG aufweist.

4. Verfahren nach Anspruch 3, wobei der Reinigungsschritt f) eine Vakuumdestillation ist, die vorzugsweise bei einem Druck zwischen 0,01 MPa und 0,07 MPa durchgeführt wird.

5. Verfahren nach einem der vorhergehenden Ansprüche, wobei vor Schritt a) eine Verdampfung der zu regenerierenden MEG-Lösung (100), die Wasser und gelöste Salze enthält, bei Umgebungsdruck in einer Anfangsverdampfungseinheit durchgeführt wird, vorzugsweise bei einer Temperatur zwischen 100°C und 155°C und einem Druck zwischen 0,1 MPa und 0,15 MPa, zum Herstellen eines Gasaustrags, der reich an Wasser ist, und eines flüssigen Rests, der reich an MEG und an Salzen ist, der komplett in Schritt a) geleitet wird.

6. Verfahren nach einem der vorhergehenden Ansprüche, wobei Schritt a) bei einer Temperatur zwischen 120°C und 155°C und einem Druck zwischen 0,01 MPa und 0,07 MPa durchgeführt wird.

7. Verfahren nach einem der vorhergehenden Ansprüche, wobei Schritt c) bei einer Temperatur zwischen 50°C und 90°C und bei Umgebungsdruck, vorzugsweise bei einem Druck zwischen 0,1 MPa und 0,15 MPa durchgeführt wird.

8. nach einem der vorhergehenden Ansprüche, wobei der zweite Anteil (110) des Stroms (108), der reich an ausgefällten Salzen ist, aus dem Behälter (1002) mit Unterbrechungen in die Einheit zur Trennung fest/flüssig (1003) geleitet wird, vorzugsweise wenn die Konzentration an anorganischen Salzen des Stroms (108), der reich an ausgefällten Salzen ist, zwischen 10 Gewichts-% und 50 Gewichts-%, vorzugsweise zwischen 15 % Gewichts-% und 30 Gewichts-% liegt.

9. Verfahren nach einem der vorhergehenden Ansprüche, wobei der Teil (114) des oder der gesamte flüssige Austrag (112), der in Schritt c) erhalten wird, mit Unterbrechungen in die Trenneinheit (1004) von Schritt d) geleitet wird.

10. Verfahren nach einem der vorhergehenden Ansprüche, wobei ein zusätzlicher flüssiger oder gasförmiger Strom (117) in die zweite Verdampfungseinheit geleitet wird, damit die gewonnene Menge MEG in dem Strom (115) in Schritt d) erhöht wird, wobei der Strom ausgewählt ist aus Wasser, Wasserdampf oder einem Inertgas.

11. Verfahren nach einem der vorhergehenden Ansprüche, wobei Schritt d) bei einer Temperatur zwischen 120°C und 155°C und einem Druck zwischen 0,01 MPa und 0,07 MPa durchgeführt wird.

## Claims

1. Process for regenerating a solution of monoethylene glycol (MEG) containing water and dissolved salts, including the following steps:
a) said solution (100) is evaporated under vacuum in a first evaporation unit (1000) to produce a gaseous effluent including MEG and water (101), and a liquid residue enriched in MEG and in salts (104), a first portion (106) of which is recycled into said first vacuum evaporation unit (1000);
b) a second portion (105) of said liquid residue enriched in MEG and in salts is optionally sent into a tank (1002) in which the temperature of said second portion (105) of said salt-enriched liquid residue is lowered so as to precipitate inorganic salts to form a stream enriched in precipitated salts (108), a first fraction (109) of which is preferably recycled into the tank (1002), and a stream depleted in precipitated salts (107) recycled into the vacuum evaporation unit (1000) in step a);
c) said second portion (105) of said liquid residue enriched in MEG and in salts (104) obtained from step a) or a second fraction (110) of said stream enriched in precipitated salts (108) obtained in step b) is sent, preferably intermittently, into a solid/liquid separation zone (1003) to separate a stream containing precipitated salts including inorganic salts (111) and a liquid effluent including dissolved salts, among which are carboxylic acid salts (112);
d) a portion (114) or all of said liquid effluent (112) obtained in step c) is sent, preferably intermittently, into a second evaporation unit (1004) different from the first vacuum evaporation unit (1000) to perform a separation between the MEG and, at least in part, the carboxylic acid salts by evaporating under vacuum the portion (114) or all of said liquid effluent (112) obtained in step c) to form a vapour enriched in MEG and depleted in carboxylic acid salts (115) and a residual liquid effluent enriched in carboxylic acid salts (116);
e) the vapour enriched in MEG and depleted in carboxylic acid salts (115) is recycled into step a), or the vapour enriched in MEG and depleted in carboxylic acid salts (115) is mixed with the gaseous effluent including MEG and water (101) obtained from step a), and said gaseous effluent including MEG and water (101) obtained from step a) or said mixture is used to produce the regenerated MEG solution.

2. Process according to Claim 1, in which, in step e), the vapour enriched in MEG and depleted in carboxylic acid salts (115) obtained from step d) is recycled into step a).

3. Process according to Claim 2, in which a step f) of purifying the gaseous effluent (101) obtained from step a) is also performed in a purification unit (1001) to form a stream of water (103) and the regenerated MEG solution (102), preferably including from 70% to 95% by weight of MEG.

4. Process according to Claim 3, in which the purification step f) is a vacuum distillation, preferably performed at a pressure of between 0.01 MPa and 0.07 MPa.

5. Process according to any one of the preceding claims, in which, prior to step a), evaporation at atmospheric pressure of the MEG solution containing water and dissolved salts (100) to be regenerated is performed in an initial evaporation unit, preferably at a temperature of between 100°C and 155°C and a pressure of between 0.1 MPa and 0.15 MPa, to produce a water-enriched gaseous effluent and a liquid residue enriched in MEG and in salts, all of which is sent into step a).

6. Process according to any one of the preceding claims, in which step a) is performed at a temperature of between 120°C and 155°C, and at a pressure of between 0.01 MPa and 0.07 MPa.

7. Process according to any one of the preceding claims, in which step c) is performed at a temperature of between 50°C and 90°C, and at atmospheric pressure, preferably at a pressure of between 0.1 MPa and 0.15 MPa.

8. Process according to any one of the preceding claims, in which the second fraction (110) of said stream enriched in precipitated salts (108) obtained from the tank (1002) is sent into the solid/liquid separation unit (1003) intermittently, preferably if the concentration of inorganic salts of said stream enriched in precipitated salts (108) is between 10% by weight and 50% by weight, preferably between 15% by weight and 30% by weight.

9. Process according to one of the preceding claims, in which the portion (114) or all of said liquid effluent (112) obtained in step c) is sent intermittently into the separation unit (1004) of step d).

10. Process according to one of the preceding claims, in which an additional liquid or gaseous stream (117) is sent into said second evaporation unit to increase the amount of MEG recovered in the stream (115) during step d), said stream being chosen from water, steam or an inert gas.

11. Process according to one of the preceding claims, in which step d) is performed at a temperature of between 120°C and 155°C, and at a pressure of between 0.01 MPa and 0.07 MPa.
